# EUROPÄISCHE PATENTSCHRIFT

(11) **EP 1 058 677 B1**
(45) Veröffentlichungstag und Bekanntmachung des Hinweises auf die Patenterteilung: **08.01.2003**
(21) Anmeldenummer: 99915533.6
(22) Anmeldetag: 23.02.1999
(51) Int. Cl.: C07C 209/00

(54) **VERFAHREN ZUR HYDRIERUNG VON ALIPHATISCHEN ALPHA, OMEGA-DINITRILEN**
METHOD FOR HYDROGENATING ALIPHATIC ALPHA-, OMEGA-DINITRILES
PROCEDE D'HYDROGENATION DE DINITRILES ALPHA, OMEGA ALIPHATIQUES

(30) Priorität: 06.03.1998 DE 19809686
(43) Veröffentlichungstag der Anmeldung: 13.12.2000
(73) Patentinhaber: BASF AKTIENGESELLSCHAFT, 67056 Ludwigshafen (DE)
(72) Erfinder: VOIT, Guido, D-67251 Freinsheim (DE); OHLBACH, Frank, D-69221 Dossenheim (DE); LUYKEN, Hermann, D-67069 Ludwigshafen (DE); MERGER, Martin, D-67227 Frankenthal (DE); REHFINGER, Alwin, D-67112 Mutterstadt (DE); FISCHER, Rolf, Hartmuth, D-69121 Heidelberg (DE); BASSLER, Peter, D-68519 Viernheim (DE); ANSMANN, Andreas, D-69168 Wiesloch (DE)
(86) Internationale Anmeldenummer: EP9901149
(87) Internationale Veröffentlichungsnummer: WO99044982

(56) Entgegenhaltungen:
- WO-A-93/16034
- WO-A-98/43941
- DE-A- 19 614 283
- DE-C- 19 630 788
- US-A- 5 512 697

## Beschreibung

Die vorliegende Erfindung betrifft ein Verfahren zur Hydrierung von aliphatischen alpha, omega-Dintrilen in Gegenwart eines heterogenen Festbett-Katalysators, dadurch gekennzeichnet, daß die Reaktionsmischung 2 µmol bis 30 mmol Ca in Form eines basischen Salzes bezogen auf 10 mol eingesetztes aliphatisches alpha, omega-Dinitril enthält.

Verfahren zur Herstellung von aliphatischen alpha, omega-Aminonitrilen oder alpha, omega-Diaminen durch Hydrierung von aliphatischen alpha, omega-Dintrilen in Gegenwart eines heterogenen Festbett-Katalysators sind allgemein bekannt.

US-A-5512697 beschreibt die Hydrierung von alpha, omega-Dinitrilen zu alpha, omega-Aminonitrilen in Gegenwart eines Katalysators und Lithiumhydroxid, Natriumhydroxid und Kaliumhydroxid.

DE-A-19630788 und DE-A-19614283 beschreiben Katalysatoren, die bei der Hydrierung einer Kohlenstoff-Stickstoff-Mehrfachbindung zu einer Aminogruppe eingesetzt werden können und die als Dotierstoffe Lithium-, Natrium-, Kalium-, Rubiolium-, Cäsium-, Magnesium-, Calcium-, Strontium- oder Barium-Verbindungen enthalten können.

Nachteilig bei diesen Verfahren ist die Entstehung größerer Mengen an unerwünschten Nebenprodukten. So entsteht insbesondere Tetrahydrazepin (THA) der Formel (I) 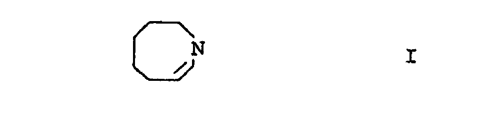 das von dem Produkt oder der produktmischung nur schlecht abtrennbar ist, bei der Hydrierung von Adipodinitril (ADN) zu einer Mischung aus 6-Aminocapronitril (ACN) und Hexamethylendiamin (HMD) in Mengen von mehr als 1000 ppm (bez. auf HMD).

Aus DE-A 44 46 894 ist bekannt bei der Hydrierung von ADN zu einer Mischung aus ACN und HMD an einem Ni-, Ru-, Rh oder Co-Katalysator insbesondere in Suspensionsfahrweise zur Erhöhung der ACN-Ausbeute der Reaktionsmischung Lithiumhydroxid zuzusetzen. Aus WO-A 93/16034 ist bekannt, bei der Hydrierung von ADN zu einer Mischung aus ACN und HMD an einem Raney-Ni-Katalysator zur Erhöhung der ACN-Ausbeute in Suspensionsfahrweise der Reaktionsmischung Lithium-, Natrium- oder Kaliumhydroxid zuzusetzen.

Nachteiligerweise erhöht sich bei der Suspensionsfahrweise an Raney-Katalysatoren die THA-Menge auf mehr als 1 Gew.-% (bez. auf HMD).

Aufgabe der vorliegenden Erfindung war es daher, ein Verfahren Hydrierung von aliphatischen alpha, omega-Dintrilen in Gegenwart eines heterogenen Festbett-Katalysators bereitzustellen, das die genannten Nachteile nicht aufweist und die Herstellung von aliphatischen alpha, omega-Aminonitrilen und/oder alpha, omega-Diaminen auf technisch einfache und wirtschaftliche Weise ermöglicht.

Demgemäß wurde das eingangs definierte Verfahren gefunden.

Als Ausgangsstoffe im erfindungsgemäßen Verfahren werden aliphatische alpha,omega-Dinitrile der allgemeinen Formel II

NC-(CH₂)ₙ-CN II

in der n eine ganze Zahl von 1 bis 10, insbesondere 2, 3, 4, 5 und 6, bedeutet, eingesetzt. Besonders bevorzugte Verbindungen I sind Bernsteinsäuredinitril, Glutarsäuredinitril, Adipinsäuredinitril ("Adiponitril"), Pimelinsäuredinitril und Korksäuredinitril ("Suberonitril"), ganz besonders bevorzugt Adiponitril.

Nach dem erfindungsgemäßen Verfahren werden die vorstehend beschriebenen Dinitrile II vorzugsweise in Gegenwart eines Lösungsmittels unter Verwendung eines hetrogenen Festbett-Katalysators partiell zu alpha,omega-Aminonitrilen der allgemeinen Formel III

NC-(CH₂)ₙ-CH₂-NH₂ III

und/oder zu alpha, omega-Diaminen der allgemeinen Formel IV H₂N-CH₂- (CH₂)n-CH₂-NH₂
hydriert, wobei n die vorstehend genannte Bedeutung hat. Besonders bevorzugte Aminonitrile III sind solche, in denen n einen Wert von 2, 3, 4, 5 oder 6 hat, insbesondere 4, d.h. 4-Aminobutansäurenitril, 5-Aminopentansäurenitril, 6-Aminohexansäurenitril ("6-Aminocapronitril"), 7-Aminoheptansäurenitril und 8-Aminooctansäurenitril, ganz besonders bevorzugt 6-Aminocapronitril.

Besonders bevorzugte Diamine IV sind solche, in denen n einen Wert von 2, 3, 4, 5 oder 6 hat, insbesondere 4, d.h. 1,4-Diaminobutan, 1,5-Diaminopentan, 1,6-Diaminohexan (HMD), 1,7-Diaminoheptan und 1,8-Diaminooctan, ganz besonders bevorzugt HMD.

Ganz besonders bevorzugt ist die ebenfalls die gleichzeitige Herstellung von ACN und HMD.

Man kann vorzugsweise die partielle Hydrierung diskontinuierlich oder kontinuierlich in einem Festbettreaktor in Riesel- oder Sumpffahrweise durchführen, wobei man üblicherweise eine Temperatur im Bereich von 20 bis 150, vorzugsweise von 30 bis 120°C und einen Druck in der Regel im Bereich von 2 bis 40, vorzugsweise von 3 bis 30 MPa wählt. Vorteilhaft kann man die partielle Hydrierung in Gegenwart eines Lösungsmittels, bevorzugt Ammoniak, Amine, Diamine und Triamine mit 1 bis 6 C-Atomen wie Trimethylamin, Triethylamin, Tripropylamin und Tributylamin oder Alkohol, bevorzugt Methanol und Ethanol, besonders bevorzugt Ammoniak durchführen. In einer bevorzugten Ausführungsform wählt man einen Gehalt an Ammoniak im Bereich von 0,5 bis 10, bevorzugt von 0,5 bis 6 g pro g Adipodinitril. Bevorzugt wählt man dabei eine Katalysatorbelastung im Bereich von 0,1 bis 2,0, vorzugsweise von 0,3 bis 1,0 kg Adipodinitril/l*h. Auch hier kann man durch Veränderung der Verweilzeit den Umsatz und damit die Selektivität gezielt einstellen.

Im Falle von Aolipoolinitril als Dinitril kann das Molverhältnis von 6-Aminocapronitril zu Hexamethylendiamin, und damit das Molverhältnis von Caprolactam zu Hexamethylendiamin, durch den jeweils gewählten Adipodinitril-Umsatz gesteuert werden. Bevorzugt arbeitet man bei Adipodinitril-Umsätzen im Bereich von 10 bis 90, bevorzugt von 30 bis 80%, um hohe 6-Aminocapronitril-Selektivitäten zu erhalten.

In der Regel liegt die Summe aus 6-Aminocapronitril und Hexamethylendiamin je nach Katalysator und Reaktionsbedingungen bei 98 bis 99%.

Erfindungsgemäß wird die Hydrierung derart durchgeführt, daß die Reaktionsmischung 2 µmol bis 30 mmol, vozugsweise 10 µmol bis 3 mmol, insbesondere 10 µmol bis 300 µmol Ca in Form eines basischen organischen, vorzugsweise anorganischen Salzes (V), wie Carbonats, vorzusweise Oxids, insbesondere Hydroxids, oder Gemische solcher Salze bezogen auf 10 mol eingesetztes aliphatisches alpha, omega-Dinitril enthält.

Besonders bevorzugt ist dabei ein solches Salz (V), das in der Reaktionsmischung vollständig löslich ist.

Ein Salz (V) kann der Reaktionsmischung vor der Hydrierung gelöst vorzugsweise in mindestens einem der Bestandteile der Reaktionsmischung oder der Reaktionsmischung in fester Form zugegeben werden. Es ist auch möglich, dem Reaktionsgemisch während der Hydrierung ein Salz (V) zuzugeben, wobei die vorteilhafte Wirkung gegenüber einer Zugabe vor der Hydrierung geringer ausfällt.

Die Hydrierung kann man an sich nach einem der bekannten Verfah-ren durchführen, indem man im allgemeinen die Hydrierung in Gegenwart von Nickel-, Cobalt-, Eisen- oder Rhodium-haltigen Katalysatoren durchführt. Dabei können die Katalysatoren als Trägerkatalysatoren oder als Vollkatalysatoren verwendet werden. Als Katalysatorträger kommen beispielsweise Aluminiumoxid, Siliciumdioxid, Titandioxid, Magnesiumoxid, Aktivkohlen und Spinelle in Frage.

In einer bevorzugten Ausführungsform hydriert man das Dinitril bei erhöhter Temperatur und erhöhtem Druck in Gegenwart eines Lösungsmittels und eines hetrogenen Festbett-Katalysators, indem man einen Katalysator verwendet, der
(a) eine Verbindung auf der Basis eines Metalles, ausgewählt aus der Gruppe, bestehend aus Nickel, Cobalt, Eisen, Ruthenium und Rhodium, enthält und
(b) von 0,01 bis 25, vorzugsweise von 0,1 bis 5 Gew.-%, bezogen auf (a), eines Promotors auf der Basis eines Metalles, ausgewählt aus der Gruppe, bestehend aus Palladium, Platin, Iridium, Osmium, Kupfer, Silber, Gold, Chrom, Molybdän, Wolfram, Mangan, Rhenium, Zink, Cadmium, Blei, Aluminium, Zinn, Phosphor, Arsen, Antimon, Bismut und Seltenerdmetalle, sowie
(c) von 0 bis 5, vorzugsweise von 0,1 bis 3 Gew.-%, bezogen auf (a), einer Verbindung auf der Basis eines Alkalimetalles oder eines Erdalkalimetalles, enthält,
mit der Maßgabe, daß, wenn als Komponente (a) eine Verbindung auf der Basis von nur Ruthenium oder Rhodium oder Ruthenium und Rhodium oder Nickel und Rhodium gewählt wird, der Promotor (b) gewünschtenfalls entfallen kann, sowie mit der weiteren Maßgabe, daß, die Komponente (a) nicht auf der Basis von Eisen besteht, wenn die Komponente (b) Aluminium ist.

Bevorzugte Katalysatoren sind solche, in denen die Komponente (a) mindestens eine Verbindung auf der Basis eines Metalles, ausgewählt aus der Gruppe aus Nickel, Cobalt und Eisen, in einer Menge im Bereich von 10 bis 95 Gew.-% sowie Ruthenium und/oder Rhodium in einer Menge im Bereich von 0,1 bis 5 Gew.-%, jeweils bezogen auf die Summe der Komponenten (a) bis (c), enthält, die Komponente (b) mindestens einen Promotor auf der Basis eines Metalles, ausgewählt aus der Gruppe bestehend aus Silber, Kupfer, Mangan, Rhenium, Blei und Phosphor, in einer Menge im Bereich von 0,1 bis 5 Gew.-%, bezogen auf (a), enthält, und
die Komponente (c) mindestens eine Verbindung auf der Basis der Alkalimetalle und Erdalkalimetalle, ausgewählt aus der Gruppe bestehend aus Lithium, Natrium, Kalium, Cäsium, Magnesium und Calcium, in einer Menge im Bereich von 0,1 bis 5 Gew.-% enthält.

Besonders bevorzugte Katalysatoren sind:
Katalysator A, enthaltend 90 Gew.-% Cobaltoxid (CoO), 5 Gew.-% Manganoxid (Mn₂O₃), 3 Gew.-% Phosphorpentoxid und 2 Gew.-% Natriumoxid (Na₂O),
Katalysator B, enthaltend 20 Gew.-% Cobaltoxid (CoO), 5 Gew.-% Manganoxid (Mn₂O₃), 0,3 Gew.-% Silberoxid (Ag₂O), 70 Gew.-% Siliciumdioxid (SiO₂), 3,5 Gew.-% Aluminiumoxid (Al₂O₃), 0,4 Gew.-% Eisenoxid (Fe₂O₃), 0,4 Gew.-% Magnesiumoxid (MgO) sowie 0,4 Gew.-% Calciumoxid (CaO), und
Katalysator C, enthaltend 20 Gew.-% Nickeloxid (NiO), 67,42 Gew.-% Siliciumdioxid (SiO₂), 3,7 Gew.-% Aluminiumoxid (Al₂O₃), 0,8 Gew.-% Eisenoxid (Fe₂O₃), 0,76 Gew.-% Magnesiumoxid (MgO), 1,92 Gew.-% Calciumoxid (CaO), 3,4 Gew.-% Natriumoxid (Na₂O) sowie 2,0 Gew.-% Kaliumoxid (K₂O).

In einer weiteren bevorzugten Ausführungsform hydriert man das Dinitril bei erhöhter Temperatur und erhöhtem Druck in Gegenwart eines Lösungsmittels und eines hetrogenen Festbett-Katalysators,
(a) metallisches Cobalt, eine Cobalt-Verbindung oder deren Gemische, wobei der Anteil an metallischem Cobalt bezogen auf (a) 20 bis 100 Gew. -% beträgt,
(b) 10 bis 70 Gew. -% bezogen auf (a) metallisches Eisen, Eisenoxid, eine weitere Eisenverbindung oder deren Gemische, wobei der Anteil an Eisenoxid bezogen auf (b) 20 bis 100 Gew. -% beträgt,
(c) 0 bis 1 Gew. -% bezogen auf die Summe aus (a) und (b) eine Verbindung auf der Basis eines Alkalimetalls, Erdalkalimetalls oder Zink.

Bevorzugt sind solche Katalysatoren, deren Anteil in dem Katalysator-Vorläufer vor der Aktivierung mit Wasserstoff oder einer Gasmischung, die Wasserstoff und ein Inertgas wie Stickstoff enthält, an einer oder mehrerer Co-Verbindungen, berechnet als Cobalt-II-oxid, 10 bis 80 Gew.-%, vorzugsweise 20 bis 70 Gew.-%, insbesondere 30 bis 60 Gew.-% beträgt.

Bevorzugt sind solche Katalysatoren, deren Anteil in dem Katalysator-Vorläufer vor der Aktivierung mit Wasserstoff oder einer Gasmischung, die Wasserstoff und ein Inertgas wie Stickstoff enthält, an einer oder mehrerer Fe-Verbindungen, berechnet als Eisen-III-oxid, 20 bis 90 Gew.-%, vorzugsweise 30 bis 80 Gew.-%, insbesondere 40 bis 70 Gew.-% beträgt.

Bei diesen Katalysatoren kann es sich um Voll- oder Trägerkatalysatoren handeln. Als Trägermaterialien kommen beispielsweise poröse Oxide wie Aluminiumoxid, Siliciumdioxid, Alumosilikate. Lanthanoxid, Titandioxid, Zirkondioxid, Magnesiumoxid, Zinkoxid und Zeolithe sowie Aktivkohle oder Mischungen davon in Betracht.

Die Herstellung erfolgt in der Regel derart, daß man einen oder mehrere Vorläufer der Komponente (a) zusammen mit Vorläufer der Komponente (b) und gewünschtenfalls mit einem oder mehrere Vorläufer der Spurenkomponente (c) in Gegenwart oder Abwesenheit von Trägermaterialien (je nachdem welcher Katalysatortyp gewünscht ist) ausfällt, gewünschtenfalls den so erhaltenen Katalysatorvorläufer zu Strängen oder Tabletten verarbeitet, trocknet und anschließend calciniert. Trägerkatalysatoren sind im allgemeinen auch erhältlich, indem man den Träger mit einer Lösung der Komponenten (a) , (b) und gewünschtenfalls (c) tränkt, wobei man die einzelnen Komponenten gleichzeitig oder nacheinander zugeben kann, oder indem man die Komponenten (a), (b) und gewünschtenfalls (c) auf den Träger nach an sich bekannten Methoden aufsprüht.

Als Vorläufer der Komponenten (a) und (b) kommen in der Regel gut wasserlösliche Salze der zuvor genannten Metalle wie Nitrate, Chloride, Acetate, Formiate und Sulfate in Betracht, vorzugsweise Nitrate.

Als Vorläufer der Komponente (c) kommen in der Regel gut wasserlösliche Salze der Alkalimetalle oder Erdalkalimetalle, wie Lithium, Natrium, Kalium, Rubidium, Cäsium, Magnesium oder Calcium, oder Zink sowie deren Gemische, wie Hydroxide, Carbonate, Nitrate, Chloride, Acetate, Formiate und Sulfate in Betracht, vorzugsweise Carbonate und Hydroxide.

Die Fällung erfolgt im allgemeinen aus wäßrigen Lösungen, wahlweise durch Zugabe von Fällungsreagenzien, durch Änderung des pH-Wertes oder durch Änderung der Temperatur.

Als Fällungsreagenzien können beispielsweise Ammoniumcarbonat oder Hydroxide oder Karbonate der Alkalimetalle eingesetzt werden. Werden Alkalimetall-Verbindungen Reagenzien eingesetzt, so empfiehlt es sich, die Niederschläge beispielsweise durch Auswaschen mit Wasser von anhaftenden Alkalimetall-Verbindungen zu befreien. Dies kann direkt nach der Abtrennung des Niederschlags von der Mutterlauge oder nach einem Trocknungs- und Calzinierschritt durchgeführt werden. Die Trocknung kann in an sich bekannter Weise, vorzugsweise in Sprühtürmen durchgeführt, wobei man den Niederschlag in der Regel in einer Flüssigkeit, vorteilhaft Wasser, aufschlämmt. Üblicherweise trocknet man die so erhaltene Katalysatormasse im allgemeinen bei Temperaturen im Bereich von 80 bis 150 °C, vorzugsweise von 80 bis 120°C vor.

Das Calcinieren nimmt man üblicherweise bei Temperaturen im Bereich von 150 bis 500°C, wobei in Einzelfällen auch Temperaturen von bis zu 1 000°C in Betracht kommen können, vorzugsweise 200 bis 450°C in einem Gasstrom aus Luft oder Stickstoff in hierfür geeigneten Apparaturen wie Horden- oder Drehrohröfen vor.

Das Pulver kann, insbesondere für den Fall, daß die Katalysatormasse in einem Festbett verwendet werden soll, zu Formkörpern, wie Strängen oder Tabletten in an sich bekannter Weise verarbeitet werden.

Bei der Herstellung von Strängen können Hilfsmittel wie anorganische Säuren, organische Säuren oder Basen wie Ammoniak zugegeben werden, wobei die Hilfsmittel Cobalt oder Eisenverbindungen enthalten können. Nach dem Verstrangen kann man die Stränge bei Temperaturen unter 200°C trocknen und bei Temperaturen im Bereich von 150 bis 500°C, wobei in Einzelfällen auch Temperaturen von bis zu 1000°C in Betracht kommen können, vorzugsweise 200 bis 450°C in einem Gasstrom aus Luft oder Stickstoff in hierfür geeigneten Apparaturen wie Horden- oder Drehrohröfen calcinieren.

Bei der Herstellung von Tabletten können organische oder anorganische Hilfsmittel wie Stearate, Graphit oder Talkum zugegeben werden.

Nach dem Calcinieren setzt man die Katalysatormasse einer reduzierenden Atmosphäre aus (,'Aktivierung"), indem sie beispielsweise bei einer Temperatur im Bereich von 150 bis 300°C, vorzugsweise von 200 bis 280°C 2 bis 96 Stunden einer Wasserstoff-Atmosphäre oder einer Gasmischung, enthaltend Wasserstoff und ein Inertgas wie Stickstoff, aussetzt. Die Katalysatorbelastung beträgt hierbei 200 bis 2000 1 pro 1 Katalysator und pro Stunde.

Vorteilhaft führt man die Aktivierung des Katalysators direkt im Synthese-Reaktor durch, da hierdurch üblicherweise ein ansonsten erforderlicher Zwischenschritt, nämlich die Passivierung der Oberfläche bei üblicherweise Temperaturen im Bereich von 20 bis 80, vorzugsweise von 25 bis 35°C mittels Sauerstoff-Stickstoff-Mischungen wie Luft, wegfällt. Die Aktivierung passivierter Katalysatoren nimmt man dann bevorzugt im Synthese-Reaktor bei einer Temperatur im Bereich von 150 bis 300, vorzugsweise von 200 bis 280°C in einer Wasserstoff-haltigen Atmosphäre vor.

Die Katalysatoren enthalten
(a) metallisches Cobalt, eine Cobalt-Verbindung oder deren Gemische, wobei der Anteil an metallischem Cobalt, bezogen auf
(a) 20 bis 100 Gew.-%, vorzugsweise 30 bis 90 Gew.-%, insbesondere 40 bis 70 Gew.-% beträgt,
(b) 10 bis 70 Gew.-% bezogen auf (a) metallisches Eisen, Eisenoxid eine weitere Eisenverbindung oder deren Gemische, wobei der Anteil an Eisenoxid, bezogen auf (b) 20 bis 100 Gew.-%, vorzugsweise 20 bis 80 Gew.-%, insbesondere 30 bis 70 Gew.-% beträgt und
(c) 0 bis 1 Gew.-%, bezogen auf die Summe aus (a) und (b) eine Verbindung auf der Basis eines Alkalimetalls, Erdalkalimetalls oder Zink.

Die Katalysatoren können als Festbettkatalysatoren in Sumpf- oder Rieselfahrweise eingesetzt werden.

Besonders bevorzugte Katalysatoren sind solche, die
a) eine Verbindung auf der Basis von Eisen wie Eisenoxid enthalten und
b) von 0 bis 5 Gew.-% bezogen auf (a) eines Promotors auf der Basis eines Elementes oder 2,3,4 oder 5 Elementen ausgewählt aus der Gruppe bestehend aus Aluminium, Silizium, Zirkonium, Vanadium und Titan sowie
c) von 0 bis 5 Gew.-%, vorzugsweise 0,1 bis 3 Gew.-%, insbesondere 0,1 bis 0,5 Gew.-% bezogen auf (a) einer Verbindung auf der Basis eines Alkali- oder Erdalkalimetalles, vorzugsweise ausgewählt aus der Gruppe bestehend aus Lithium, Natrium, Kalium, Rubidium, Cäsium, Magnesium und Calcium.

Bei den bevorzugt einsetzbaren Katalysatoren kann es sich um Voll- oder Trägerkatalysatoren handeln. Als Trägermaterialien kommen beispielsweise poröse Oxide wie Aluminiumoxid, Siliciumdioxid, Alumosilikate, Lanthanoxid, Titandioxid, Zirkondioxid, Magnesiumoxid, Zinkoxid und Zeolithe sowie Aktivkohle oder Mischungen davon in Betracht.

Die Herstellung erfolgt in der Regel derart, daß man Vorläufer der Komponenten (a) zusammen mit Vorläufern der Promotoren (Komponenten (b) und gewünschtenfalls mit Vorläufern der Spurenkomponenten (c) in Gegenwart oder Abwesenheit von Trägermaterialien (je nachdem welcher Katalysatortyp gewünscht ist) ausfällt, gewünschtenfalls den so erhaltenen Katalysatorvorläufer zu Strängen oder Tabletten verarbeitet, trocknet und anschließend calciniert. Trägerkatalysatoren sind im allgemeinen auch erhältlich, indem man den Träger mit einer Lösung der Komponenten (a), (b) und gewünschtenfalls (c) tränkt, wobei man die einzelnen Komponenten gleichzeitig oder nacheinander zugeben kann, oder indem man die Komponenten (a), (b) und gewünschtenfalls (c) auf den Träger nach an sich bekannten Methoden aufsprüht.

Als Vorläufer der Komponenten (a) kommen in der Regel gut wasserlösliche Salze der zuvor genannten Metalle wie Nitrate, Chloride, Acetate, Formiate und Sulfate in Betracht, vorzugsweise Nitrate.

Als Vorläufer der Komponenten (b) kommen in der Regel gut wasserlösliche Salze oder Komplexsalze der zuvor genannten Metalle wie Nitrate, Chloride, Acetate, Formiate und Sulfate sowie insbesondere Hexachloroplatinat in Betracht, vorzugsweise Nitrate und Hexachloroplatinat.

Als Vorläufer der Komponenten (c) kommen in der Regel gut wasserlösliche Salze der zuvor genannten Alkalimetalle und Erdalkalimetalle wie Hydroxide, Carbonate, Nitrate, Chloride, Acetate, Formiate und Sulfate in Betracht, vorzugsweise Hydroxide und Carbonate.

Die Fällung erfolgt im allgemeinen aus wäßrigen Lösungen, wahlweise durch Zugabe von Fällungsreagenzien, durch Änderung des pH-Wertes oder durch Änderung der Temperatur.

üblicherweise trocknet man die so erhaltene Katalysatorvormasse im allgemeinen bei Temperaturen im Bereich von 80 bis 150, vorzugsweise von 80 bis 120°C vor.

Das Calcinieren nimmt man üblicherweise bei Temperaturen im Bereich von 150 bis 500, vorzugsweise von 200 bis 450°C in einem Gasstrom aus Luft oder Stickstoff vor.

Nach dem Calcinieren setzt man die erhaltene Katalysatormasse im allgemeinen einer reduzierenden Atmosphäre aus ("Aktivierung"), beispielsweise indem man sie bei einer Temperatur im Bereich von 80 bis 250, vorzugsweise von 80 bis 180°C bei Katalysatoren auf der Basis von Ruthenium oder Rhodium als Komponente (a), oder im Bereich von 200 bis 500, vorzugsweise von 250 bis 400°C bei Katalysatoren auf der Basis eines der Metalle ausgewählt aus der Gruppe aus Nickel, Cobalt und Eisen als Komponente (a) 2 bis 24 h einer Wasserstoff-Atmosphäre oder einer Gasmischung, enthaltend Wasserstoff und ein Inertgas wie Stickstoff, aussetzt. Die Katalysatarbelastung beträgt hierbei bevorzugt 200 1 pro 1 Katalysator.

Vorteilhaft führt man die Aktivierung des Katalysators direkt im Synthese-Reaktor durch, da hierdurch üblicherweise ein ansonsten erforderlicher Zwischenschritt, nämlich die Passivierung der Oberfläche bei üblicherweise Temperaturen im Bereich von 20 bis 80, vorzugsweise von 25 bis 35°C mittels Sauerstoff-Stickstoff-Mischungen wie Luft, wegfällt. Die Aktivierung passivierter Katalysatoren nimmt man dann bevorzugt im Synthese-Reaktor bei einer Temperatur im Bereich von 180 bis 500, vorzugsweise von 200 bis 350°C in einer Wasserstoff-haltigen Atmosphäre vor.

Die Katalysatoren können als Festbettkatalysatoren in Sumpf- oder Rieselfahrweise eingesetzt werden.

Nach dem erfindungsgemäßen Verfahren erhält man alpha,omega-Aminonitrile und/oder alpha, omega Diamine in in guten Selektivitäten und mit nur geringen Mengen an unerwünschten Nebenprodukten. Alpha, omega-Aminonitrile und alpha, omega Diamine sind wichtige Ausgangsverbindungen zur Herstellung von cyclischen Lactamen, insbesondere 6-Aminocapronitril für Caprolactam und HMD.

### Beispiele

### a) Katalysatorherstellung

Die Katalysatoren wurden hergestellt durch 6-stündiges Tempern eines Magnetiterzes bei 1500°C unter Stickstoff. Das verwendete Magnetiterz hatte folgende Zusammensetzung:
72 Gew.-% Fe
0,07 Gew.-% Al
0,03 Gew.-% Ca
0,04 Gew.-% Mg
0,11 Gew.-% Si
0,01 Gew.-% Ti
Rest Sauerstoff

Der abgekühlte Schmelzblock wurde im Backenbrecher zerkleinert und eine Siebfraktion der Teilchengröße 3-6 mm ausgesiebt. Der oxidische Katalysator wurde im Wasserstoff/Stickstoff-Strom bei 450°C 72 h reduziert und anschließend in einem Stickstoff/Luft-Strom (24 h mit 1 Vol.-% Luft in Stickstoff) bei unter 45°C passiviert.

### b) Hydrierung

### Vergleichsbeispiel

Ein Rohrreaktor (Länge 180 cm, Durchmesser 30 mm) wurde mit 740 ml (1816 g) der nach (a) hergestellten Katalysator-Masse befüllt und bei 150 bar im Wasserstoff-Strom (500 Nl/h) reduziert. Dabei wurde die Temperatur innerhalb von 24 h von 30°C auf 340°C angehoben und anschließend 72 h bei 340°C gehalten.

Nach Absenken der Temperatur wurde dem Reaktor bei 250 bar ein Gemisch aus 400 ml/h ADN, 770 g/h Ammoniak und 500 Nl/h Wasserstoff zugeführt. Bei 100°C betrug der ADN-Umsatz 70%, doie ACN-Selektivität betrug 60% und die Gesamtselektivität von ACN und HMD lag bei 99%.

Der THA-Gehalt bezogen auf HMD im Hydrieraustrag lag bei 1200 ppm.

### Beispiel 1

Es wurde wie im Vergleichsbeispiel verfahren mit der Ausnahme, daß dem ADN 9,62 µmol CaO bezogen auf 10 mol ADN zugesetzt wurden.

Der THA-Gehalt bezogen auf HMD im Hydrieraustrag lag bei 400 ppm.

### Beispiel 2

Es wurde wie im Vergleichsbeispiel verfahren mit der Ausnahme, daß dem ADN 72,9 µmol Ca(OH)₂ bezogen auf 10 mol ADN zugesetzt wurden.

Der THA-Gehalt bezogen auf HMD im Hydrieraustrag lag bei 300 ppm.

## Patentansprüche

1. Verfahren zur Hydrierung von aliphatischen alpha, omega-Dintrilen in Gegenwart eines heterogenen Festbett-Katalysators, **dadurch gekennzeichnet, daß** die Reaktionsmischung 2 µmol bis 30 mmol Ca in Form eines basischen Salzes bezogen auf 10 mol eingesetztes aliphatisches alpha, omega-Dinitril enthält.

2. Verfahren nach Anspruch 1 zur Herstellung von aliphatischen alpha, omega-Aminonitrilen.

3. Verfahren nach Anspruch 1 zur Herstellung von aliphatischen alpha, omega-Diaminen.

4. Verfahren nach den Ansprüchen 1 bis 3, wobei man einen heterogenen Katalysator, dessen aktive Komponente auf Eisen, Cobalt, Nickel, Rhodium oder Ruthenium oder deren Verbindungen oder deren Gemische basiert, einsetzt.

5. Verfahren nach den Ansprüchen 1 bis 3, wobei man einen heterogenen Katalysator, dessen aktive Komponente auf Eisen basiert, einsetzt.

6. Verfahren nach den Ansprüchen 1 bis 5, wobei der Katalysator ein Trägerkatalysator ist.

7. Verfahren nach den Ansprüchen 1 bis 5, wobei der Katalysator ein Vollkatalysator ist.

8. Verfahren nach den Ansprüchen 1 bis 7, wobei man als Dinitril Adipodinitril einsetzt unter Erhalt von 6-Aminocapronitril.

9. Verfahren nach den Ansprüchen 1 bis 7, wobei man als Dinitril Adipodinitril einsetzt unter Erhalt von Hexamethylendiamin.

10. Verfahren nach den Ansprüchen 1 bis 7 zur Herstellung einer Mischung enthaltend 6-Aminocapronitril und Hexamethylendiamin ausgehend von Adipodinitril.

11. Verfahren nach Anspruch 10 zur gleichzeitigen Herstellung von 6-Aminocapronitril und Hexamethylendiamin ausgehend von Adipodinitril durch
(1) partielle Hydrierung von Adipodinitril gemäß einem Verfahren nach den Ansprüchen 1 bis 4 unter Erhalt einer Mischung enthaltend 6-Aminocapronitril, Hexamethylendiamin und Adipodinitril und
(2) Abtrennung von 6-Aminocapronitril und Hexamethylendiamin aus der Mischung.

## Claims

1. A process for hydrogenation of aliphatic alpha, omega-dinitriles in the presence of a heterogeneous fixed bed catalyst, which comprises using a reaction mixture comprising from 2 µmol to 30 mmol Ca in the form of a basic salt, based on 10 mol of aliphatic alpha, omega-dinitrile used.

2. A process as claimed in claim 1 for preparing aliphatic alpha, omega-aminonitriles.

3. A process as claimed in claim 1 for preparing aliphatic alpha, omega-diamines.

4. A process as claimed in any of claims 1 to 3, wherein the heterogeneous catalyst used comprises an active component based on iron, cobalt, nickel, rhodium or ruthenium or their compounds or their mixtures.

5. A process as claimed in any of claims 1 to 3, wherein the heterogeneous catalyst used comprises an active component based on iron.

6. A process as claimed in any of claims 1 to 5, wherein the catalyst is a supported catalyst.

7. A process as claimed in any of claims 1 to 5, wherein the catalyst is an unsupported catalyst.

8. A process as claimed in any of claims 1 to 7, wherein the dinitrile used is adiponitrile to obtain 6-aminocapronitrile.

9. A process as claimed in any of claims 1 to 7, wherein the dinitrile used is adiponitrile to obtain hexamethylenediamine.

10. A process as claimed in any of claims 1 to 7 for preparing a mixture comprising 6-aminocapronitrile and hexamethylenediamine starting from adiponitrile.

11. A process as claimed in claim 10 for coproduction of 6-aminocapronitrile and hexamethylenediamine starting from adiponitrile by
(1) partial hydrogenation of adiponitrile as per a process as claimed in any of claims 1 to 4 to obtain a mixture comprising 6-aminocapronitrile, hexamethylenediamine and adipodinitrile and
(2) removing 6-aminocapronitrile and hexamethylenediamine from the mixture.

## Revendications

1. Procédé d'hydrogénation d'α,ω-dinitriles aliphatiques en présence d'un catalyseur hétérogène à lit fixe, **caractérisé en ce que** le mélange réactionnel contient de 2 µmoles à 30 mmoles de Ca sous la forme d'un sel basique, par rapport à 10 moles d'α,ω-dinitrile aliphatique mises en oeuvre.

2. Procédé selon la revendication 1 pour la préparation d'α,ω-aminonitriles aliphatiques.

3. Procédé selon la revendication 1 pour la préparation d'α,ω-diamines aliphatiques.

4. Procédé selon les revendications 1 à 3, dans lequel on met en oeuvre un catalyseur hétérogène dont le composant actif est à base de fer, cobalt, nickel, rhodium ou ruthénium, ou leurs mélanges.

5. Procédé selon les revendications 1 à 3, dans lequel on met en oeuvre un catalyseur hétérogène dont le composant actif est à base de fer.

6. Procédé selon les revendications 1 à 5, où le catalyseur est un catalyseur sur support.

7. Procédé selon les revendications 1 à 5, où le catalyseur est un catalyseur plein.

8. Procédé selon les revendications 1 à 7, où l'on met en oeuvre en tant que dinitrile de l'adiponitrile, avec obtention de 6-aminocapronitrile.

9. Procédé selon les revendications 1 à 7, où l'on met en oeuvre en tant que dinitrile de l'adiponitrile, avec obtention d'hexaméthylènediamine.

10. Procédé selon les revendications 1 à 7 pour la préparation d'un mélange contenant du 6-aminocapronitrile et de l'hexaméthylènediamine à partir d'adiponitrile.

11. Procédé selon la revendication 10 pour la préparation simultanée de 6-aminocapronitrile et d'hexaméthylénediamine à partir d'adiponitrile par
(1) hydrogénation partielle d'adiponitrile par un procédé selon les revendications 1 à 4, avec obtention d'un mélange contenant du 6-aminocapronitrile, de l'hexaméthylènediamine et de l'adiponitrile et
(2) séparation du 6-aminocapronitrile et de l'hexaméthylènediamine du mélange.
